# EUROPEAN PATENT APPLICATION

(11) **EP 1 821 483 A1**
(43) Date of publication of application: **22.08.2007**
(21) Application number: 06003509.4
(22) Date of filing: 21.02.2006
(51) Int. Cl.: H04L 29/06, G06F 19/00

(54) **Computer network system and method for operating the network system screenshot and sourceshot control**

(71) Applicant: BrainLAB AG, 85551 Kirchheim/Heimstetten (DE)
(72) Inventor: Bauch, Thomas, 85232 Bergkirchen (DE); Lang, Gerhard, 81245 München (DE); Krüger, Peter, 85551 Kirchheim (DE)
(74) Representative: Schwabe - Sandmair - Marx

(57) **Abstract**

The invention relates to Computer network system, comprising a controlling PC; at least one controlled PC which is controlled by the controlling PC via a data connection including a network; and at least one central terminal having a display characterized by a video router which is connected to each of the at least one controlled PCs and at least one central terminal display to transmit video or monitor signals from each of the controlled PCs to at least one central terminal display as well as to a method for taking multiple screenshots and/or sourceshots of a selection from a plurality of images from a plurality of image sources, wherein the shots are initiated simultaneously while keeping at least 80 % of the original size and/or resolution of each image and a plurality of image files is formed containing the plurality of images having the at least 80 % of the original size and/or resolution.

## Description

The present invention concerns a computer network system, especially a network connection to connect several electronically controlled medical devices while providing a central terminal and control display which can easily be handled and kept sterile.

The invention concerns further a method for taking screenshots and sourceshots.

It is known from the Microsoft Windows remote desktop system that the desktop of one PC is transferred over the network to a second PC. On the second PC a user can use a keyboard and a mouse to control the first PC. This input is also transferred over the network back to the first PC.

Furthermore, a Keyboard-Video-Mouse switch (KVM-switch) is known, wherein a central monitor, keyboard and a mouse are connected to the KVM. Two or more PCs are connected to the KVM. The KVM is used to switch between the different PCs. Every connected PC can alternatively be seen and controlled over the central monitor/keyboard/mouse. The video and input signals are directly transferred over their normal cables, such as RGB-cable or PS/2-cable.

EP 1 433 432 A1 corresponding to US 2004/0116908 A1 discloses a device for coupling at least two medically applicable instruments, which are coupled to at least two control apparatuses, and includes a central control unit coupled to input or output connections of the at least two control apparatuses. The central control unit includes at least one processor that receives output signals from the at least two control apparatuses and converts the output signals into a unified format. At least one processor receives inputted control signals, converts the control signals into formats corresponding to the respective at least two control apparatuses, and transfers the converted control signals to the at least two control apparatuses to control the at least two medically applicable instruments.

US 5,788,688 discloses a surgeon's command and control system including an independent personal computer based electronic control unit that unifies various pieces of equipment currently found in an endoscopic surgical suite into a surgeon centred system. The system utilizes programmed software which simplifies equipment management tasks that currently encumber the surgeon and operating room staff. The surgeon's command and control hardware centers around the personal computer communicating with a sterile control panel located at the surgeon's operating station. A frame store card serves as an electronic pallet to compose and superimpose graphic images onto a surgical image transmitted from an endoscopic camera for display on a heads-up display monitor and the surgical operating station.

US 6,117,127 discloses a medical system work station for open or minimally invasive surgery which has a holder tray and at least one terminal unit for handheld instruments of one or more medical devices, and at least one equipment center, spatially separated from the terminal unit, for accepting non-manipulated components of the medical devices, and at least one connection unit that connects the terminal unit and the equipment centre with one another.

US 6,928,490 B1 discloses a networking infrastructure for an operating room, comprising a plurality of medical devices, wherein each device is connected through a single communication channel to the network and each device may be controlled through a local interface, or through a remote interface available through the network. Furthermore, the networking infrastructure operates in robust manner with respect to the removal of a communication channel to the network associated with the removal of medical device from the network, or with respect to the addition of a communication channel to the network associated with the addition of a medical device to the network.

It is an object of the invention to provide a computer network system and a method for operating the same which allows medical personnel to work with and control several medical devices without impairing the performance of these devices.

This object is solved by the computer network system and the method as defined in the independent claims. Preferred embodiments are defined in the dependent claims.

A computer network system comprises at least one and preferably two or more controlled computers, which are hereinafter designated as controlled PCs, and which can include a software, control or computer system for controlling a respective device, preferably a medical device, which can also be or is controlled by another and preferably remote controlling computer or PC, which is connected to the controlled PC over a network. In general, a network according to the present invention can include every data connection between two computers or PCs and can be a standard network or any other data connection such as e.g. serial or parallel data connection or cables. The computer network system comprises further a controlling computer hereinafter referred to as a controlling PC, which is connected via the network, as e.g. the Ethernet, to the controlled PCs to transmit control commands from the controlling PC to the controlled PCs. Furthermore, at least one central terminal display is provided, which is connected and preferably directly connected to the controlling PC. According to the invention, preferably a single video routing hardware is provided which is preferably connected to each controlled PC to receive a monitor or video signal from each controlled PC and which is furthermore connected to each central terminal display to transmit a monitor or video signal preferably of the same quality and resolution as that received from one or more of the controlled PCs to the central terminal display.

Thus, by using different channels for video and input data, the invention combines the advantages of the KVM and remote desktop technologies. The video routing using a video router instead of transmitting the video signals over the network provides superior image quality and performance. The input transfer over a data connection provides a broad compatibility of different devices that cannot be achieved by using a KVM system. By separating the network or data transfer system into a network system for transferring commands on the one hand and into a video router for transmitting monitor or video signals on the other hand, the original image quality and resolution provided by each of the controlled PCs which can be the control devices of medical instruments, such as imaging devices or laparoscopic devices, can be retained and is not impaired while being transmitted to the central terminal display.

In case of using a touch screen as central terminal it is even possible to control different devices with incompatible touch screen drivers, because the central touch screen is never directly connected to the controlled PC, but to a controlling PC which can convert command signals from the central terminal touch screen into respective different command signals of the controlled PCs.

It is also possible to simultaneously control a PC from different terminals. The video routing hardware can distribute the video signal to more than one terminal and all these terminals can display and operate the same PC simultaneously.

The possibility to control different medical devices from one or more terminals eliminates the need for the surgeon or assisting personal to manipulate and control each single device through its own control screen, buttons or switches, so that it is not necessary to access the respective medical device which can be placed at a remote location from the surgeon or assisting personal, so that the time-consuming and sometimes cumbersome procedure for manipulating and controlling each separate medical device is no longer necessary. Further, it may also allow to completely eliminating the screen and control means of the controlled PC, which simplifies the system set-up.

According to the invention, medical devices that do not offer an option for sterile manipulation and that commonly require handling by a non-sterile assistant can be controlled in the sterile field via the central touch screen which can be provided with a sterile draping.

Since according to the present invention the original graphic user interface of the respective medical device can be presented to the user, since the original video or monitor signal is directly and preferably unchanged sent to the central terminal display, the user does not have to adapt to an additional or foreign user interface, which enhances the usability and operability of the whole computer network system. Therefore, it is not necessary to implement an own mirrored user interface for every single controlled application. The controlled application does not need to support the additional control in any way, since it is, according to the invention, not necessary to distinguish whether some input has its origin in a local keyboard, mouse or touch screen, or whether it was generated by e.g. an additional software which can be installed on each respective controlled PC called "input client".

Preferably error or warning messages from at least one and preferably all integrated controlled PCs can be displayed on the central terminal, so that the user only has to watch a central terminal display and is not forced to take a look on every single screen of every separate medical device or to switch through all devices to see if errors occurred or warnings were output. If such an error or warning message occurs, this message can be always visible on a central terminal, e.g. by displaying it in front of the actual screen, regardless which medical device or PC is currently visible there.

According to a further aspect, the invention relates to a method for operating a network system as described above, wherein control commands from a controlling PC or controlling computer are sent via a data connection which can include a network to at least one controlled computer or PC. According to the invention, the video or monitor signals from at least one and preferably all controlled computers or PCs are sent to a video router or video routing hardware, which transmits these monitor or video signals to at least one central terminal screen without using the data connection or network used for transmitting control commands between the controlling computer and the controlled computers.

Furthermore, the invention relates to a computer program, which can be loaded or running on a computer and which, when loaded or running on a computer, performs the method for operating the network system as described in this application.

The invention relates also to a program storage medium or computer program product comprising such a computer program.

Thus, the invention allows controlling different PCs over one or more central terminals. These PCs can be - but don't have to be - part of medical devices. The PCs can be operated via touch screen or keyboard and mouse. On the controlled PCs an additional software called "Input Client" can be installed that performs the control of that PC by generating operating system input events in a similar way like for instance touch screen drivers generate input events. This Input Client can also display a small graphical user interface in front of all other applications on that PC. On the central terminal, the graphical user interface of the Input client can be used to switch between different controlled PCs. On the local displays of the controlled PCs, the graphical user interface of the Input Clients can display status and warning messages. Since this user interface may occlude some part of the applications user interface, it detects which application is currently visible and moves itself to a suitable position on the screen, where it does not handicap the operation of that application.

The Input Client can e.g. detect warning messages of the controlled applications and can distribute them to the Input Clients of all other integrated PCs, who then can display a warning to the user. The detection of such warnings from an application can be done through inter-application communication, by searching operating system data structures to identify whether there is for example a dialog window with the text "error" or "warning" in its title or by pattern recognition to identify warnings or errors by analyzing the content that is displayed by the application.

The invention allows thus controlling one or more PCs from a central terminal (e.g. touch screen or monitor / keyboard / mouse), wherein to control such a PC only the input (keyboard, mouse / touch clicks) can be transferred e.g. over a network. Alternatively to the network, any other data connections to the controlled PCs can be used, e.g. serial or parallel cables. On the controlled PC additional software, the Input Client, can receive the input events and can translate these into normal operating system input events. The principle can be compared with a touch screen driver that generates input events from the data that it receives over the serial connection to the touch screen.

The monitor signal of the controlled PCs can be distributed by a video routing hardware (e.g. a matrix switch, video processor, ...) to the central terminal. So the operator will see the original monitor content like he would see when he uses a KVM, but he controls the devices over a data connection that is independent from the video connection.

The input signals from the central terminals (e.g. PS/2 cables of keyboard and mouse or serial / USB cable from touch screen) are permanently connected or transmitted to the controlling PC. The monitor input of the central monitor or touch screen is connected to the video routing hardware. The controlling PC does not need to display any content on its own graphics output and even does not need to have one.

The operator will see the original image of the controlled PC when he operates the system. His input is received by the controlling PC and then transferred over the data connection to the controlled PC, where the control data is translated by the Input Client into system input events. When the operator switches from one controlled PC to another one, the controlling PC issues a switch command to the video routing hardware. The video routing hardware will then route the video signal of the newly controlled PC instead of the signal of the formerly controlled PC to the central terminal. The controlling PC will transfer the following input to the Input Client of the newly controlled PC.

To initiate such a switching between different controlled PCs different mechanisms can be used:
- a graphical user interface of the Input Client
- a wireless remote control that sends switching commands to the controlling PC
- a cable-bound remote control that sends switching commands to the controlling PC
- switch buttons that are embedded into the monitor housing and connected to the controlling PC
- hot keys for keyboard control.

The invention will be described by way of examples and preferred embodiments with reference to the following figures which show:
- Figure 1: a first embodiment of a computer network system using monitor/keyboard/mouse terminals;
- Figure 2: a second embodiment using touch screens; and
- Figure 3: a third embodiment using in combination monitor/keyboard/mouse terminals and touch screens.

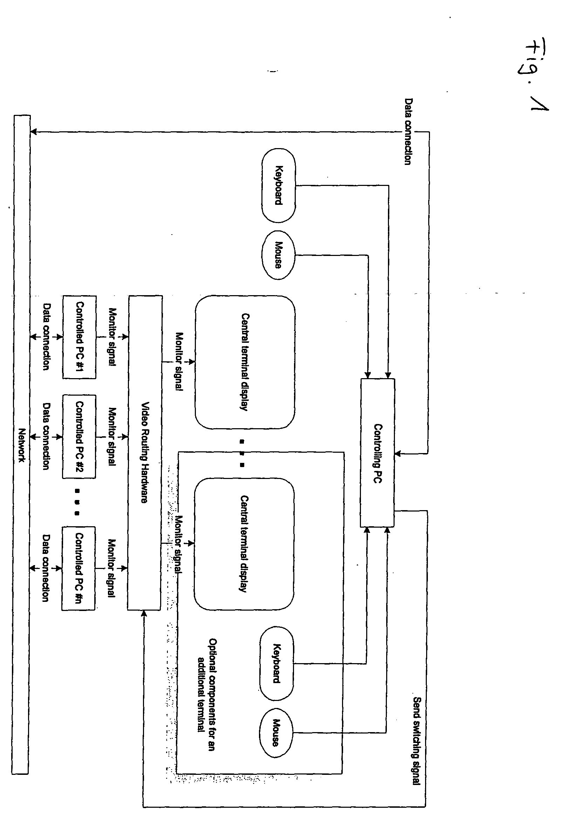

Figure 1 shows as a first embodiment an example set up using a network connection to transfer input commands input using the keyboard and/or mouse to then controlled PCs. In this embodiment the central terminal consists of a monitor as central terminal display, a keyboard and a mouse. For a second terminal an additional keyboard and mouse could be connected to the controlling PC and an additional monitor can be connected to the video routing hardware. Each one of the controlled PCs can have a local monitor/keyboard/mouse or touch screen, which are not shown in the Figure.

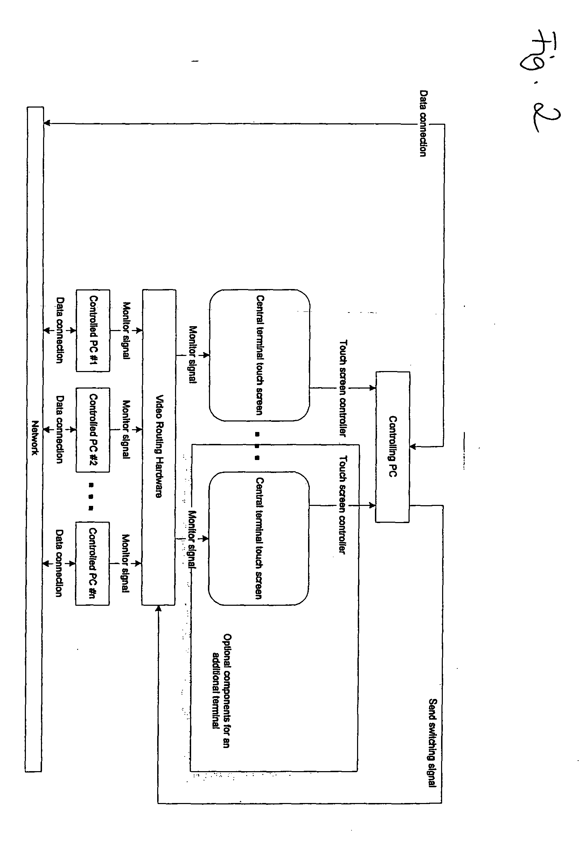

Figure 2 shows a second embodiment using touch screens instead of monitor/keyboard/mouse for the central terminals. The monitor signal for the touch screens is provided by the video routing hardware and the touch input using the central terminal touch screen is processed by the controlling PC and sent via the data connection and the network to the respective controlled PCs.

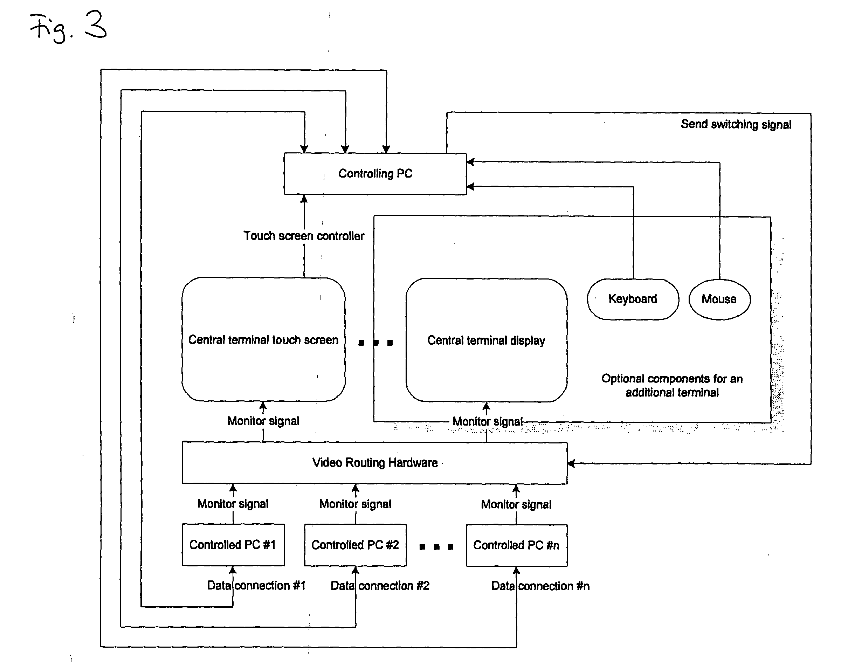

Figure 3 shows a third embodiment using monitor/keyboard/mouse terminals and touch screen terminals in combination, which can also be used at the same time.

Furthermore, an alternative wiring is shown, wherein other data connections, such as for example serial cables, are used instead of a network to transfer the input data from the central terminal touch screen, keyboard or mouse to the respective controlled PCs.

The Input Client can display a small graphical user interface (GUI) in front of all other applications. This GUI will then occlude some part of the application that the user wants to control. Since the occluded part can be important for the proper function of that software, the GUI can be moved to different positions on the screen. Every different application that shall be controlled can have a different position on the screen where the GUI of the Input Client does not handicap the operation of that applications and where it does not cover important information displayed by the application. Such suitable positions can be configured for all applications that shall be controlled. The Input Client will automatically detect which application on a controlled PC is currently visible and moves itself accordingly to a position that is suitable for that application.

The Input Client is able to detect warning or error messages on the controlled PC. This detection can be achieved in different ways. One possibility is an inter-application-communication. In this case the application that originates the warnings is aware of the Input Client and supports the inter-application-communication. It will then directly inform the Input Client of the warning.

A second possibility to detect warnings are operating system data structures of the currently existing windows. The Input Client will detect every new window that is opened and will analyze whether the window displays some warning or error information. This can be achieved for example by testing whether the window title contains the word "error" or "warning".

A third possibility to detect warning or error conditions is a pattern recognition. The Input Client periodically analyzes the screen content of its PC. If a content is recognized that is known as a warning / error condition, the Input Client can detect and forward such a condition. The Client can be trained by the user which graphical content represents such conditions.

When a warning or error condition was recognized, the Input Client distributes that message through a central server on the controlling PC to all connected Input Clients on the other controlled PCs. When such an other Input Client is notified about such a condition, it can display a warning on its screen. Because all warnings from all integrated devices will be displayed by all Input Clients, a warning is always visible on all displays, including the central terminal. The user is therefore informed about warnings from any integrated device, regardless which PC is currently shown on the central terminal.

There can be different levels how critical or important a warning or an error can be. This allows the user to configure whether he wants to be informed about all warnings and errors detected by the Input Clients or whether he only wants to be informed about really serious problems.

For taking screenshots the state-of-the-art technology provides only for the possibility of taking single screenshots. In case there is more than one monitor connected to a computer or a PC, Microsoft Windows would take one large screenshot that shows all displays together and that may contain black areas when the respective screens have different resolutions.

According to an aspect, the invention relates to a method for taking screenshots and/or sourceshots of a plurality of images from a plurality of image sources, wherein the shots are initiated simultaneously e.g. by a single action of a user while keeping preferably the original resolution and/or size of each image. Thus, the invention allows taking screenshots of multiple screens at the same time and to take image stills, also called sourceshots, of a video source or a computer or PC source, which can also be possible before this image is shown on a display. According to the invention screenshots and sourceshots can also be combined and can be taken or initiated simultaneously. According to the invention a combined screenshot and/or sourceshot consisiting of at least two screenshots or at least two sourceshots or at least one screenshot and one sourceshot is formed, wherein at least 70 % and preferably more, as e.g. 80 % or 90% of the original resolution and/or size and/or color depth of each image or of the respective image source can be maintained, although these separate images are combined to a single screen- and sourceshot which can comprise a plurality of different image files.

According to a first embodiment, screenshots of multiple screens can be taken at the same time. Additionally, the invention allows selecting or changing the respective screens conveniently via a central graphical user interface or a video routing hardware, as described above. The user obtains multiple screenshots from different images sources, computers or PCs that have been taken simultaneously, as for example exactly at the same time. Within the meaning of the present application, simultaneously can also mean that the time difference between the screenshot and the corresponding sourceshot has a predetermined given maximum difference, such as for example three frames. Obtaining multiple screenshots at exactly the same time allows an exact alignment of the respective different screenshots.

Preferably it can selectively be chosen which screens shall be captured and optionally be stored, wherein every screen can be stored in a separate image file that has the resolution of the respective screen, so that there are no unused or black areas in the combined shot, even if the respective original images have different screen resolutions.

If one or more sourceshots are used as image sources, a shot of a video or PC signal can be taken before it is scaled and displayed at the screen. The user can for example display four downscaled sources on one screen which does not contain any black areas, while the pictures of all sources can be provided with the original resolution.

If screenshots and sourceshots are combined, the user can automatically be provided with an overview picture and the contained sources in full resolution.

Preferably, the sourceshot functionality also allows to capture a source that is not displayed at all on a screen, which functionality can for example be used to automatically create image captures of sources for documentation purposes in regular intervals. This automatic documentation is also possible when the respective displays show some other content and can be used for documenting a medical procedure.

A system for performing the inventive method comprises the video processor to which video sources, like for example microscopes, endoscopes and computer or PC sources, like for example a navigation system or C-arms, can be connected. Additionally, the video processors can provide or have connections to some displays like for example monitors or beamers.

The signal from a connected source can be digitized inside an input card, if necessary, i.e. if the signal from the connected source is for example an analog signal. The input card can then feed the digitized signal to a proprietary high-speed bus. Then this signal is received by output cards and displayed on a monitor.

The term "screenshot" as used in the description can be understood as being a shot performed by or from an output card. The term "sourceshot" can be understood as a shot being performed by or from an input card. Every output card can perform screenshots simultaneously with other output cards or screenshots. If there were for example 10 output cards, wherein for example every card is a dual head card with two independent monitor connectors, a simultaneous screenshot would represent up to 20 pictures at once.

Every input card can perform sourceshots simultaneously with the others. If there were for example 10 input cards and every card is a dual head card with two independent PC input connections, a simultaneous sourceshot would represent up to 20 pictures at once. Additionally, there are input cards which can connect twelve video sources instead of two PC sources, so that the number of pictures would increase accordingly.

Both, screenshot and sourceshot, can be done simultaneously, wherein a time difference between a screenshot and the corresponding sourceshot(s) of a given number of for example three frames can be interpreted as being simultaneously, as mentioned above.

## Claims

1. Computer network system, comprising:
a) a controlling PC;
b) at least one controlled PC which is controlled by the controlling PC via a data connection including a network; and
c) at least one central terminal having a display;
**characterized by**
d) a video router which is connected to each of the at least one controlled PCs and at least one central terminal display to transmit video or monitor signals from each of the controlled PCs to at least one central terminal display.

2. Computer network system according to claim 1, wherein the video router is separated from the controlling PC.

3. Computer network system according to one of the preceding claims, wherein a means of user input is a touch screen connected to the controlling PC and integrated into the central terminal display

4. Computer network system according to one of the preceding claims, wherein a means of user input is at least one keyboard and/or at least one mouse connected to the controlling PC.

5. Computer network system according to one of the preceding claims, wherein at least one of the controlled PCs is connected to or a part of a medical device.

6. Computer network system according to one of the preceding claims with several central terminals, from which different controlled PCs can be controlled simultaneously and independently.

7. Computer network system according to one of the preceding claims with several central terminals, from which one specific controlled PC can be controlled from more than one central terminal.

8. Method for operating the network system as claimed in one of the preceding claims, wherein control commands are transmitted from the controlling PC to the at least one controlled PC via a network and video signals are transmitted from the at least one controlled PC to the central terminal display via a video routing hardware which is separate from and preferably not connected to the network.

9. Method according to the preceding claim, wherein a software is installed on the at least one controlled PC to perform the control of the controlled PC by generating operating system input events out of the input events generated at the central terminal display being a touch screen or controlling PC being connected to input devices, especially mouse or keyboard.

10. Method according to any of the two preceding claims, wherein a graphical user interface is displayed in front of all other applications on the central terminal display to provide a control on the central terminal display for controlling each of the at least one controlled PCs.

11. Method according to the previous claim, wherein the graphical user interface is moved to a different position on the central terminal screen, if the part covered by the graphical user interface is designated as being an important part for the function of the software controlling the respective controlled PC.

12. Method according to any of the four previous claims, wherein a warning and/or error message transmitted via the network to the controlling PC is detected and displayed on the central terminal display and/or on displays of the controlled PCs.

13. Method according to the previous claim, wherein the detection of the warning and/or error message is based on pattern recognition.

14. Method according to any of the six previous claims, wherein the original user interface of the controlled PC is transferred to the central terminal display to maintain the screen content of the controlled PC unamended.

15. Method according to any of the seven previous claims, wherein the controlled PC reacts on an output of another controlled PC, which is transferred over the network.

16. Method according to one of the eight previous claims, in which conflicts arising from user input from several central terminals are avoided by indicating user input activity at one central terminal on the other central terminals through the graphical user interface and/or by blocking user input activity at the other central terminals for a specific period of time.

17. Method according to one of the nine preceding claims, wherein the video routing hardware distributes a video signal to more than one central terminal and all these terminals can display and operate the same controlled PC simultaneously.

18. Computer program product, which, when loaded or running on a computer, performs the method of any of the ten preceding claims.

19. Program storage medium or computer program product comprising the program of the previous claim.

20. Method for taking multiple screenshots and/or sourceshots of a selection from a plurality of images from a plurality of image sources, wherein the shots are initiated simultaneously while keeping at least 80 % of the original size and/or resolution of each image and a plurality of image files is formed containing the plurality of images having the at least 80 % of the original size and/or resolution.

21. Method according to the previous claim, wherein an image source is a display device, a monitor, a video source, a microscope, an endoscope, a C-arm, a computer, a PC, a graphic card or a navigation system.

22. Method according to one of the two preceding claims, wherein one or more screens and/or sources are selected via a central graphical user interface.

23. Method according to one of the three preceding claims, wherein every captured image is stored in a separate image file while keeping at least 80 % of the original resolution and/or size of the respective image.

24. Method according to one of the four preceding claims, wherein the screenshots and/or sourceshots are displayed as a picture-in-picture view.

25. Method for documenting a medical procedure using the method for taking a screenshot and/or a sourceshot as claimed in one of the five previous claims.

26. Method for documenting a medical procedure according to the previous claim,
wherein screenshots and/or sourceshots are taken automatically in predetermined or regular intervals.

27. Computer program, which, when loaded or running on a computer, performs the method of any of the seven preceding claims.

28. Program storage medium or computer program product comprising the program of the previous claim.
